(19) Europäisches Patentamt
European Patent Office
Office européen des brevets

(11) Publication number: **0 105 672 B1**

(12) # EUROPEAN PATENT SPECIFICATION

(45) Date of publication of patent specification: **09.04.86**

(51) Int. Cl.$^4$: **C 07 C 143/46,** C 07 C 139/00

(21) Application number: **83305621.1**

(22) Date of filing: **22.09.83**

(54) **Synthesis of sulphonated organic compounds.**

(30) Priority: **28.09.82 GB 8227675**
**27.04.83 GB 8311497**

(43) Date of publication of application:
**18.04.84 Bulletin 84/16**

(45) Publication of the grant of the patent:
**09.04.86 Bulletin 86/15**

(84) Designated Contracting States:
**AT BE CH DE FR GB IT LI NL SE**

(56) References cited:
**GB-A- 864 798**

(73) Proprietor: **THE PROCTER & GAMBLE COMPANY**
**301 East Sixth Street**
**Cincinnati Ohio 45201 (US)**
(84) **BE CH DE FR IT LI NL SE AT**

(73) Proprietor: **Procter & Gamble Limited**
**Hedley House**
**Gosforth Newcastle upon Tyne NE99 1EE (GB)**
(84) **GB**

(72) Inventor: **Hardy, Frederick Edward**
**38 Park Drive Melton Park**
**Gosforth Newcastle upon Tyne NE3 5BQ (GB)**

(74) Representative: **Gibson, Tony Nicholas et al**
**Procter & Gamble (NTC) Limited Whitley Road**
**Longbenton Newcastle upon Tyne NE12 9TS**
**(GB)**

**EP 0 105 672 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

Field of the Invention

This invention relates to the transesterification of phenol esters and more particularly to a method of transesterifying a sulphonated phenol ester. Sulphonated phenol esters comprise a class of materials suitable for use in detergent compositions as so-called bleach activators, more correctly characterised as organic peroxy acid precursors. These precursors react in aqueous solution with the inorganic peroxygen bleaches such as perborate or percarbonate normally incorporated in detergent compositions, to form organic peroxy acids which are much more effective bleaches than the inorganic peroxy salts at low temperatures (<70°C).

Background of the Invention

The conventional method of synthesising the sulphonate phenol esters involves the acylation in a solvent such as dioxan or dichlorethane of a phenol sulphonate salt with an acyl halide, (normally the chloride), eliminating hydrogen halide. This method, however, suffers from several disadvantages. The starting acyl halide is itself a difficult and unpleasant material to handle in bulk, and the equipment needed to withstand corrosive attack by the hydrogen halide byproduct of the reaction is expensive. A further problem arises in the elimination of the hydrogen halide from the reaction system. This is normally carried out by means of an inert gas sparging system but the tendency of the suspended solid reaction product to cause foaming of the reaction mixture, and the need to avoid removal of the solvent and/or the acyl halide, restricts the gas flow rate, and thus the completeness of removal. If HCl is not removed efficiently there is competition with the sulphonate group for sodium ions leading to the formation both of sodium chloride and a less stable sulphonic acid form of the product. Finally, the carrying out of the reaction on a large scale appears to have an adverse effect on the completeness of reaction and conversions in excess of 70% are difficult to obtain.

An alternative route to the synthesis of sulphonated phenol esters, particularly those having longer chain acyl groups is transesterification. This is a well established method of exchanging ester groupings between organic compounds and can be achieved by alcoholysis, acidolysis or ester-ester interchange. Alcoholysis in which an —OH group-containing compound is reacted with a lower alkyl ester having the desired acyl group, is the most frequently used technique. Acidolysis is less commonly used and involves reaction between a compound bearing a lower alkyl ester group and the appropriate carboxylic acid. Ester exchange, as the term suggests, is merely an exchange of ester groups between compounds.

All of these reactions are conventionally catalysed and a wide range of acid and base catalysts both homogeneous and heterogeneous in type are disclosed by the prior art as being of general applicability although a number of reaction systems have been found to be catalyst specific.

The transesterification of phenol esters is normally carried out by alcoholysis, but the use of this procedure for sulphonated phenol esters leads to difficulties. Firstly, the sulphonated derivatives are hydrophilic and are therefore insoluble in the relatively non-polar reaction media such as alcohols and hydrocarbons used in such transesterification reactions.

Secondly, the presence of a sulphonate group tends to deactivate the —OH group of the phenol, adversely affecting the reaction kinetics and making it difficult to achieve high reaction completeness because of the increased risk of side reactions and product degradation. The use of higher temperatures to increase the rate of reaction merely exacerbates these problems, leading to extensive carbonisation of the reaction mixture.

Ester-exchange is also unsuccessful with sulphonated phenol esters as the lower alkyl radical on the other ester in the reaction is not easily displaced under the conditions of the reaction.

Acidolysis has been proposed as an effective transesterification technique in the formation of aryl esters of aromatic acids and a disclosure of its use in the preparation of phenyl and cresyl esters of aromatic dicarboxylic acids, particularly isophthalic and terephthalic acid is given in British Patent Specification No. 1,009,484. This specification also teaches the in situ preparation of a lower acyl aromatic ester as a starting material for the transesterification reaction.

Nevertheless the procedure disclosed in BP Specification No. 1,009,484 itself gives rise to difficulties in that the catalysts generally taught therein as being suitable are relatively specific, being recited as effective in certain cases and not in others. A further disadvantage arises from the use of certain of the suitable metal-containing catalysts such as butyl-titanate and tin stearate recited in 1,009,484, namely their tendency to be retained in the reaction product, necessitating further work-up stages to achieve complete removal. The principal application of the reaction product of the process of the present invention is as an organic peroxyacid precursor, i.e. a material converted into an organic peroxy acid upon reaction with an alkaline source of hydrogen peroxide. The presence of metal ions in such a precursor material adversely affects the stability of the resultant peroxy acid and may reduce the percentage yield of the peroxy acid from the precursor-hydrogen peroxide reaction.

However, the Applicants have now found that surprisingly, transesterification of $C_2$—$C_3$ acyloxybenzene sulphonates can be carried out to a high degree of completion without any catalyst being required. The presence of a catalyst does improve the kinetics of the process but is not essential to initiate the reaction.

Whilst not wishing to be bound by any theory as to the reaction mechanism responsible for this finding it is believed that the use of an acidolysis technique for the transesterification of these phenol esters overcomes the problem of deactivation encountered in alcoholysis of the parent phenol because the electron withdrawing character of the sulphonate group enhances the tendency of the acyl group of the ester to cleave from the aromatic nucleus. The Applicants have also found that the transesterification reaction can be carried out without having to employ a solvent that serves no other purpose than providing a reaction medium. The use of the carboxylic acid reactant as the reaction medium is believed to improve the solubility of both the starting and product sulphonated ester materials insofar as it is more polar than other solvent materials such as esters. Nevertheless, a predicted disadvantage of the use of the carboxylic acid reactant as solvent is the possibility of its reaction with the acyloxybenzene sulphonate product to form the alkanoic anhydride, thereby reducing the yield and purity of the product. Surprisingly, however, the acyloxybenzene sulphonate product has not been found to react with the carboxylic acid solvent under the conditions found to be satisfactory for the process, and the reasons for this are not fully understood. Furthermore, in certain preferred embodiments, it has been found that the reaction mixture, comprising the $C_6$—$C_{18}$ acyl oxybenzene sulphonate salt together with carboxylic acid, can be processed directly into a form suitable for use in a detergent product, without an intermediate solvent extraction step to isolate the acyl oxybenzene sulphonate salt *per se*.

Summary of the Invention

Accordingly the present invention provides a method of preparing a $C_6$—$C_{18}$ acyloxybenzene sulphonate salt comprising the steps of

a) forming and maintaining a uniformly dispersed mixture of a $C_2$—$C_3$ acyloxybenzene sulphonate salt in a liquid $C_6$—$C_{18}$ substituted or unsubstituted aliphatic carboxylic acid medium, the molar ratio of carboxylic acid to sulphonate salt being in excess of 1:1 and the sulphonate salt being in particulate form;

b) raising the temperature of the mixture to a value sufficient to cause an acidolysis reaction to take place and to allow the volatilisation and removal of byproduct $C_2$—$C_3$ alkanoic acid; and

c) recovering the $C_6$—$C_{18}$ acyloxybenzene sulphonate salt.

In a preferred embodiment of the invention the method comprises:

a) forming a mixture of a phenol sulphonate salt, and a $C_2$—$C_3$ alkanoic anhydride in a liquid substituted or unsubstituted $C_6$—$C_{12}$ aliphatic carboxylic acid medium, the molar ratio of carboxylic acid to sulphonate salt being in excess of 2:1, the molar ratio of anhydride to sulphonate salt being in excess of 1:1, and the sulphonate salt being in particulate form;

b) heating the mixture under reflux to a temperature in the range 140—180°C until the phenol sulphonate salt has reacted to form a $C_2$—$C_3$ acyloxy benzene sulphonate salt;

c) raising the temperature of the mixture to a value sufficient to permit acidolysis to take place, and to allow volatilisation and removal of byproduct $C_2$—$C_3$ alkanoic acid; and

d) recovering the $C_6$—$C_{12}$ acyl oxybenzene sulphonate salt.

In a highly preferred embodiment of the process of the invention, a basic catalyst, preferably sodium acetate, is added to the reaction mixture.

Detailed Description of the Invention

The present invention comprises a method of forming a $C_6$—$C_{18}$ acyl oxybenzene sulphonate salt without the use of hazardous and corrosive halogenated starting materials and in which elevated reaction temperatures, in order to achieve high conversions, are unnecessary. Further advantages are that contamination of the finished product by catalytic metals and the use of solvents in the reaction steps are avoided. In the preferred mode of carrying out the invention, a 'one-pot' procedure is employed thereby providing further simplification of the process.

The invention is applicable to the preparation of $C_6$—$C_{18}$ acyl oxybenzene sulphonates in which the hydrocarbyl portion of the acyl group is an acyclic linear or branched moiety, or is an aliphatic moiety containing a ring substituent which may be alicyclic or aromatic in character. An example of the latter in carboxylic acid form is 3-phenyl propionic acid (hydrocinnamic acid).

One embodiment of the invention is in the preparation of linear or substantially linear $C_6$—$C_{12}$ acyl oxybenzene sulphonates, particularly linear or substantially linear $C_8$—$C_{10}$ acyl oxybenzene sulphonates. "Substantially linear", in the context of the present specification, shall be taken to refer to a hydrocarbyl moiety having no more than approximately 25% methyl branching. A preferred source of linear $C_8$—$C_{10}$ acyl groups is the light fraction of coconut fatty acid composed principally of a mixture of $C_8$ and $C_{10}$ fatty acids.

However, in a preferred embodiment of the invention the $C_6$—$C_{18}$ aliphatic group of the carboxylic acid is a non linear moiety that has a linear alkyl chain of five or more carbon atoms extending from and including the carboxyl carbon. More preferred embodiments of such non linear $C_6$—$C_{18}$ aliphatic carboxylic acids incorporate branching in at least the 2- and/or 3-position with respect to the carbonyl carbon atom and in highly preferred embodiments the acids contain from 8 to 10 carbon atoms. This embodiment of the process of the invention is particularly suitable for preparing the compounds disclosed and claimed in the Applicants' copending British Application No. 8304990 filed on February 23, 1983.

The process of the present invention can be illustrated in its broadest form by the reaction of the

sodium salt of acetyl oxybenzene sulphonate with $C_9$ aliphatic carboxylic acid to form sodium $C_9$ acyl oxybenzene sulphonate.

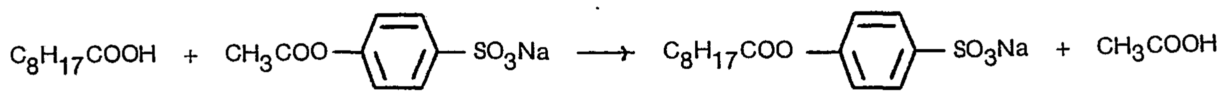

The sodium acetoxy benzene sulphonate can be prepared separately by methods known in the art e.g. by the technique disclosed in Example 1 of BP 864,798. Alternatively and preferably, the sodium acetoxy benzene sulphonate is prepared in situ by a method described hereinafter.

Although the cation is preferably sodium, other alkali metals, such as potassium and lithium, and alkali earth metals such as magnesium, can also be used. In preparing the lower acyloxybenzene sulphonate salt, propionic anhydride can also be used in place of acetic anhydride although the latter is preferred for reasons of availability, cost, reactivity and ease of processing.

In the process according to the broadest aspect of the invention the alkali or alkali earth metal $C_2$—$C_3$ acyloxybenzene sulphonate in finely divided particulate form is dispersed in liquid $C_6$—$C_{18}$ aliphatic carboxylic acid, the molar ratio of acid to salt being in excess of 1:1. The carboxylic acid temperature is raised to a value in the range 190°—240°C and the mixture is agitated, preferably together with inert gas sparging, to facilitate reaction and to enable the $C_2$—$C_3$ alkanoic acid byproduct, together with at least part of the $C_6$—$C_{18}$ aliphatic carboxylic acid to be volatilised and removed by distillation. The extent of removal of the $C_6$—$C_{18}$ aliphatic carboxylic acid at this stage is very dependent on the chain length of the acid, little removal occurring for acids of carbon chain length $>C_{12}$ unless the volatilisation is carried out under reduced pressure. However, the tendency of components of the reaction mixture that have a detergent functionality to foam under distillation conditions is aggravated by the use of vacuum so that this is not a preferred technique. Acid medium removal is also influenced by the structure of the acids, as the generally lower vapourisation temperature of non linear acids relative to linear acids of the same carbon number will result in greater removal of the former at a given reaction mix temperature.

When the reaction is complete, as evidenced by the cessation of distillate flow, the mixture is cooled to a temperature below 100°C and is then subjected to further processing to make the $C_6$—$C_{18}$ acyloxybenzene sulphonate salt suitable for incorporation into a detergent product.

In one aspect of the invention the reaction product is removed from the reaction vessel and dispersed in a hydrophobic solvent, in order to precipitate the $C_6$—$C_{18}$ acyloxybenzene sulphonate salt. This is then filtered from the supernatant liquor containing the $C_6$—$C_{18}$ aliphatic carboxylic acid and dried. In this aspect of the invention the liquor itself is then heated to volatilise the hydrophobic solvent and separate it from the excess $C_6$—$C_{18}$ aliphatic carboxylic acid. Both solvent and carboxylic acid can then be recycled back to their respective points of use in the process.

In another preferred aspect of the invention in which the aliphatic carboxylic acid is a non linear $C_6$—$C_{12}$ material and a significant proportion of the unreacted acid has been removed by volatilisation, a meltable organic carrier material is added to the cooled, but still fluid, reaction product, the addition taking place under an atmosphere of an inert gas such as nitrogen in order to avoid product degradation. The temperature of this mixture is then adjusted to permit the mixture to be mechanically worked into particulates such as flakes, granules, noodles or prills, before being cooled to ambient temperature.

Reaction mixtures in which linear aliphatic $C_6$—$C_{12}$ carboxylic acids have been used as the reaction medium can also be utilised directly, as described above, to form particulates. However, the use of such mixtures in this manner is less advantageous when a bland odour is an important requirement for the reaction product in particulate form. This is because even small amounts of linear carboxylic acid (e.g. 1% on an acyl oxybenzene sulphonate salt basis) give the reaction product a pungent odour which is difficult to mask when the product is incorporated into a detergent composition. The odour can be prevented by the addition of a sufficient amount of a suitable base (e.g. sodium carbonate) to saponify the residual linear carboxylic acid. In highly preferred embodiments of the invention, in which sodium acetate is employed as a catalyst in the acidolysis reaction the residual carboxylic acid will be associated with the sodium ion from the catalyst and the addition of a base to saponify the acid and hence remove the odour, may be unnecessary. This odour problem does not arise with the use of non linear aliphatic carboxylic acids having the preferred structure disclosed hereinafter.

One advantage of the process of the present invention is the elimination from the reaction mixture of conventional solvents whose only function is to serve as a suspending medium for the reactants. Instead, one of the reactants, viz. the $C_6$—$C_{18}$ aliphatic carboxylic acid is itself used as a reaction medium but this requires that it be in a liquid phase, preferably having a low viscosity. Aliphatic carboxylic acids containing 12 or more carbon atoms are solid at ambient temperaturs and therefore need to be heated in order to provide a liquid medium into which the $C_2$—$C_3$ acyloxy benzene sulphonate can be dispersed, but $C_6$—$C_{10}$ aliphatic carboxylic acids are liquids at ambient temperatures, thus facilitating the dispersion of the solid phenol sulphonate.

The aliphatic carboxylic acids suitable for use in the process of the present invention can be linear or

predominantly linear (as hereinbefore defined) such acids being derived from natural oils and fats, or for the substantially linear materials, from petroleum hydrocarbon feedstocks using OXO or Ziegler synthesis techniques.

However, the preferred aliphatic carboxylic acids are non linear in structure, containing at least five carbon atoms in a linear chain extending from and including the carbonyl carbon. The effects of structure on the hydrophobicity of organic compounds as represented by their partition coefficients between octanol and water are described by A. Leo et al in Chemical Reviews, *71*, pp 525—616 (1971). The authors provide numerical values for the change in $LogP_{OCT}$ (where $P_{OCT}$ is the partition coefficient between octanol and water) associated with the incorporation of various substituents into a range of structures. This permits a value for $LogP_{OCT}$ to be predicted for any structure, and on this basis preferred non linear carboxylic acids are those having a $logP_{OCT}$ in the range from 1.9 to 4.1.

The non linearity may take the form of aliphatic branching or of aromatic or cycloaliphatic structures. It is preferred that there be branching on at least the 2- and/or 3-carbon atom with respect to the carbonyl carbon but 2,2-, branching is not preferred because of the adverse effect of such configuration on the perhydrolysis performance of the acyloxybenzene sulphonate salt made therefrom.

Highly preferred aliphatic carboxylic acids contain from 7 to 9 carbon atoms and examples of non linear acids of this type are 2-ethyl hexanoic acid, 3,5,5-trimethyl hexanoic acid and 3-phenyl propionic acid.

The amount of $C_6$—$C_{18}$ carboxylic acid necessary to maintain the reaction mixture as a stirrable fluid is a function of the physical characteristics of the system, such as the temperature dependence of viscosity of the acid and the crystalline form of the phenol sulphonate starting material and product. However, it has been found highly desirable to have a starting molar ratio of $C_6$—$C_{18}$ carboxylic acid to phenol sulphonate of at least 2:1 so as to maintain adequate fluidity throughout the reaction and a molar ratio of at least 3:1 is preferred, the most preferred molar ratio being in the range from 4:1 to 6:1. The use of molar ratios in excess of 6:1, whilst providing more fluid reaction mixtures is not believed to be advantageous in that the risk of back reaction between the $C_6$—$C_{18}$ carboxylic acid and the $C_6$—$C_{18}$ acyloxybenzene sulphonate product to form the $C_6$—$C_{18}$ alkanoic anhydride is increased. In addition, larger reaction volumes have to be handled and more carboxylic acid has to be recovered and recycled to the reaction.

The phenol sulphonate salt has only slight solubility in the alkanoic acid and must be finely divided in order to maximise the surface area available for reaction. Preferably the material should be less than 100 microns in particle size and ideally should be as fine as possible. These criteria are met in the preferred process in which the $C_2$—$C_3$ acyloxybenzene sulphonate is formed in situ and exists as a very finely divided solid of particle size <50 microns. However, where the $C_2$—$C_3$ acyl oxybenzene sulphonate is produced in a separate location, conventional comminution techniques such as colloid milling of a suspension of the solid can be used to achieve the desired particle size.

The temperature of the dispersion is raised to 80°—100°C to initiate the reaction and the reactor must be fitted with an effective agitation system in order to maintain the uniformity of the dispersion throughout the reaction period. However, as the reaction proceeds, the viscosity of the reaction mixture increases progressively with the increase in solids:liquids ratio, and removal of byproduct $C_2$—$C_3$ alkanoic acid becomes more difficult as raising the temperature of the reaction mixture to reduce its viscosity can lead to local overheating and consequent colour degradation. The design of the agitation system therefore has to accommodate both a progressive increase in solids content throughout the reaction at the same time as the maintenance of efficient mixing to ensure the release of volatilised byproduct acid.

Removal of the byproduct $C_2$—$C_3$ alkanoic acid is assisted by inert gas sparging which also serves to prevent oxidative degradation of the reaction mixture. The gas can be any of those conventionally used for this purpose with nitrogen being the most preferred. The temperature of the reaction is allowed to increase to a value in the range from 190°C to 240°C and progress of the reaction is monitored by the rate of removal of byproduct alkanoic acid together with at least part of the unreacted excess $C_6$—$C_{18}$ carboxylic acid.

In processes in which the aliphatic carboxylic acid reaction medium contains no more than about 12 carbon atoms approximately 90% of the unreacted acid can be volatilised and removed during the reaction period. Most of the acid removal occurs towards the end of the period when the reaction is substantially complete and the temperature of the reaction mixture rises after removal of the byproduct $C_2$—$C_3$ alkanoic acid.

When this has ceased, the reaction product is allowed to cool to a temperature below 100°C before being further processed.

For reactions utilising a linear carboxylic acid, the reaction product is preferably redispersed in a solvent in which the fatty acid reaction medium is soluble and the $C_6$—$C_{18}$ acyloxybenzene sulphonate salt is insoluble. The temperature to which the reaction mixture is cooled and the solvent are chosen such that the reaction mixture is sufficiently fluid to be handled and the solvent is below its boiling point. Suitable solvents are hexane, diethyl ether, dichlorethane, and aromatic materials such as toluene or xylene.

The $C_6$—$C_{18}$ acyloxybenzene sulphonate solid is then removed by filtration while the filtrate is itself treated with e.g. activated charcoal to remove colour bodies. Finally the hydrophobic solvent is separated from the fatty acid by evaporation and both solvent and fatty acid are recycled to their respective points of use in the process.

In those embodiments of the process of the invention in which the bulk of the unreacted aliphatic

carboxylic acid reaction medium has been volatilised (viz. embodiments utilising aliphatic carboxylic acids containing 6—12 carbon atoms, more preferably those containing 7—9 carbon atoms and particularly those employing $C_7$—$C_9$ non linear aliphatic carboxylic acids), the cooled reaction product can be mixed with an organic binder material and formed into a particulate without further purification. Binders such as a normally solid nonionic surfactant, a polyethylene glycol, a fatty acid, certain anionic surfactants, a film-forming polymer or a mixture of any of these, can be used. A binder melting point of at least 40°C is required in order to provide storage stability to the particulates, but it is preferred that the binder melting point should be less than 80°C and preferably should lie in the range 50—70°C.

One group of nonionic surfactants suitable as binder materials is constituted by primary or secondary $C_8$—$C_{18}$ alcohol ethoxylates containing from 20 to 100 moles of ethylene oxide per mole of alcohol specific examples being tallow alcohol ethoxylates condensed with an average of 22 and 80 moles of ethylene oxidymole. An unethoxylated nonionic surfactant which is a particularly preferred binder material is a glyceryl mono $C_{10}$—$C_{14}$ fatty acid ester as disclosed in the Applicants' copending British Application No. 8323127 filed on August 27 1983. Suitable polyethylene glycols are those of molecular weight 2000 to 15,000, more preferably from 4000 to 8000. Suitable fatty acids are the $C_{12}$—$C_{18}$ fatty acids, preferably used in admixture with polyethylene glycols or nonionic surfactants. Anionic surfactants include soaps, alpha-sulphonated fatty acid salts, alkyl glyceryl ether sulphonates, alkyl ethoxy sulphates containing up to 10 moles of ethylene oxide per mole of alcohol and alkyl benzene sulphonates, the last named normally being used in admixture with another binder material.

The binder can be added to the reaction product after it has been removed from the reaction vessel or it can be added directly to the vessel itself. In the latter preferred technique, the reaction product is maintained as a liquid and the binder is also added in liquid form under an inert gas atmosphere. In all cases however, the formation of an intimate mixture of binder and reaction product, prior to forming the particulate, is essential. A preferred method of making such a particulate is disclosed and claimed in the US Application of Stanley Y Chung and Gianfranco L Spadini, USSN 441,978, filed November 15, 1982.

In a preferred embodiment of the process of the invention the $C_2$—$C_3$ acyloxybenzene sulphonate is formed in the same reactor as is used to effect the transesterification reaction and all of the ingredients for both reactions are added together. Thus the sodium phenol sulphonate and the $C_2$—$C_3$ alkanoic anhydride (normally in about 20% molar excess) are added to the liquid $C_6$—$C_{18}$ aliphatic carboxylic acid (present in an amount of 5× molar amount of phenol sulphonate) and the system heated under reflux at a reactor temperature in the range 160°C—175°C for 1—3 hours. Reflux is then discontinued and the reactor temperature is allowed to rise over a period of 45—90 minutes to a value in the range 200°C—220°C, during which time excess unreacted $C_2$—$C_3$ alkanoic anhydride and byproduct $C_2$—$C_3$ alkanoic acid are removed, after which the product and reaction medium separation and recovery are carried out as previously described.

The transesterification process of the invention has been described without reference to the catalysts conventionally used in such reactions. The Applicants have surprisingly found that an added catalyst is not essential for the carrying out of the process of the invention and that high yields of product of good colour can be obtained under the above conditions without the use of a catalyst.

Nevertheless, the use of catalysts, of both acidic and basic types, is believed to enhance the kinetics of the reaction.

Suitable catalysts include butyl titanate, toluene sulphonic acid, metallic magnesium (in powder form), sodium methoxide and sodium acetate. However, in certain cases, catalyst retention in the $C_6$—$C_{18}$ acyl oxybenzene sulphonate product gives rise to problems in the use of the latter for peroxyacid generation. In particular, titanium at no more than 200 ppm on a product basis causes excessive decomposition of the peroxy acid formed from the precursor. Catalytic contaminants such as titanium can be removed by additional work-up stages but this is undesirable from a manufacturing standpoint because of the increase in processing complexity and cost.

Accordingly basic catalysts are preferred, such as magnesium and sodium, although the use of methanol solutions of the latter requires a greater excess of $C_2$—$C_3$ alkanoic anhydride in the preferred 'one-pot' reaction. The most preferred catalyst is the alkali metal salt of a weak acid, particularly an alkali metal alkyl carboxylate salt. Most preferred salts are those of fatty acids having high volatility such as sodium acetate, propionate or nonanoate as these can be easily eliminated from the system thereby avoiding contamination of the product. Any added catalyst is employed in an amount of from 0.5% to 5% of the weight of the solid sulphonate component and is added therewith when the particulate dispersion is formed.

The process of the invention can be illustrated by the following Examples in which a $C_9$ aliphatic carboxylic acid is reacted with sodium phenol sulphonate and acetic anhydride to form sodium $C_9$ acyl oxybenzene sulphonate.

Example 1

One stage uncatalysed preparation of sodium nonanoyloxybenzene sulphonate

A 500 ml three necked reaction flask was charged with 79.61 g nonanoic acid (0.5 mole), 19.62 g anhydrous sodium phenol sulphonate (0.1 mole) and 12.25 g acetic anhydride (0.12 mole). The flask was fitted with a heating jacket, an agitator, a nitrogen inlet and a heated and lagged 40 cm Vigreaux column

provided with an overhead condenser and distillate collection vessel. After being charged the flask was flushed with nitrogen, a nitrogen supply was connected to the top of the Vigreaux column and heating and agitation of the reaction mixture commenced. The temperature of the mixture reached 130°C after 30 minutes and 170°C after 40 minutes at which point a frothy head appeared on the reaction mixture which had assumed a pale yellow colour. The system was then operated under reflux with an average flask temperature of 172.5°C for a period of 115 minutes after which time the frothy head disappeared. The nitrogen feed to the top of the Vigreaux column was disconnected, the column heater was switched on and nitrogen was passed into the reaction flask to assist in distillation. The reaction mixture temperature was allowed to rise to a maximum of 216°C over a period of 70 minutes during which time 22.98 g of a distillate composed of acetic acid and nonanoic acid was collected. GLC analysis of the distillate showed that there was no acetic anhydride present. The residual contents of the reaction flask, a pale yellow waxy paste, were allowed to cool overnight to ambient temperature and were then dispersed in 1 litre of diethyl ether and filtered. This procedure was repeated twice and the final product was dried in a vacuum dessicator and ground to a fine powder. The final yield was 28.74 g of a white solid, the NMR analysis of which gave (by weight):

| | |
|---|---|
| 80.3% | Sodium nonanoyloxybenzene sulphonate |
| 16.3% | Sodium acetoxybenzene sulphonate |
| 3.4% | Sodium phenol sulphonate |

the overall yield of sodium nonanoyloxybenzene sulphonate being 69% of theoretical.

### Example 2

One stage preparation of sodium nonanoyl oxybenzene sulphonate using toluene sulphonic acid catalyst

A similar procedure and apparatus was used to that described above with the following differences. 1 g of p-toluene sulphonic acid was added to the flask contents before the start of the reaction and the reflux stage took 95 minutes at a flask temperature of 170°C. The distillation stage took 50 minutes and the maximum flask temperature was 202°C. Total yield from the reaction was 28 g of a grey solid, the NMR analysis of which showed (by weight):

| | |
|---|---|
| 94.8% | Sodium nonanoyloxybenzene sulphonate |
| 3.6% | Sodium p-toluene sulphonate |
| 1.6% | Sodium phenol sulphonate |

The overall yield was 83.1% of theoretical.

### Example 3

One stage preparation of sodium nonanoyloxybenzene sulphonate using magnesium powder

The procedure and apparatus were as in the earlier preparations. 0.1 g magnesium powder was added to the flask before reaction commenced and a reflux temperature of 167°C was used for 115 minutes. Distillation took 65 minutes with a maximum reaction mix temperature of 215°C. 30.4 g of white solid product was obtained containing 0.11% Mg (by Atomic Absorption) and NMR analysis showed (by weight):

| | |
|---|---|
| 91.4% | Sodium nonanoyl oxybenzene sulphonate |
| 2.7% | Sodium acetoxy benzene sulphonate |
| 5.9% | Sodium phenol sulphonate |

The overall yield was 82.7% of theoretical.

### Example 4

One stage preparation of sodium nonanoyl oxybenzene sulphonate using sodium acetate

The procedure and apparatus were as before and 1 g of sodium acetate was employed as a catalyst. Refluxing took 140 minutes at 166°C and distillation took 65 minutes with a maximum flask temperature of 218°C. A white product was obtained weighing 32.7 g, the NMR analysis of which showed:

| | |
|---|---|
| 99% | Sodium nonanoyl oxybenzene sulphonate |
| 1% | Sodium acetate |

The overall yield was 96.3% of theoretical.

From these experiments it can be seen that the process of the invention does not require catalysis in order to obtain satisfactory yields of material of acceptable purity. Optimisation of the reaction conditions can be expected to improve upon the yield and purity obtained using this procedure. The experiments also show that acidic and basic catalysis can provide a reduction in reaction time and/or temperature. However, acidic catalysts tend to produce poorer product colour and, as previously stated, catalyst metal contamination of the product can adversely affect the stability of the organic peroxy acid produced by perhydrolysis with inorganic peroxygen bleaches. Basic catalysts do not suffer from the same disadvantages although the use of metal alkoxides increases ingredient costs by requiring a greater excess of acetic anhydride. The preferred catalyst is a sodium salt of a weak acid such as a carboxylate as this minimises contamination of the product, and does not increase the quantities of reactants necessary to carry out the process.

Example 5

One stage preparation of sodium 3,5,5 trimethyl hexanoyl oxybenzene sulphonate using sodium acetate catalyst

A similar procedure and apparatus to that used in Example 1 was employed with the following quantities of reactants:

| | |
|---|---|
| 3,5,5, trimethyl hexanoic acid (isononanoic acid) | 127.85 g (0.81 mole) |
| Acetic anhydride | 36.75 g (0.36 mole) |
| Sodium phenol sulphonate | 58.85 g (0.3 mole) |
| Sodium acetate (anhydrous) | 3.0 g |

This mixture provides a fluid suspension. The reaction flask contents were heated to 160°C and refluxed under a nitrogen atmosphere for 3¾ hrs before the apparatus was converted into a distillation mode and the flask temperature allowed to rise to 260°C over a period of 45 minutes during which time 59.63 g of a mixture of isononanoic acid, acetic acid and a trace of acetic anhydride were collected in the distillate receivers. During this time the contents of the reaction flask changed from a creamy white fluid suspension to a thick foaming pale yellow mass that was difficult to keep agitated. The heating was turned off and, whilst maintaining a nitrogen atmosphere in the flask, 40 g $TAE_{80}$ was added to the flask which improved the fluidity of the contents. The flask was allowed to cool and after a further 45 minutes the temperature of the reaction flask contents had fallen to 70°C at which stage the flask was emptied and the mixture allowed to cool to room temperature. 156.83 g of reaction product in the form of an off-white friable particulate was obtained having the following analysis:

| | |
|---|---|
| Sodium 3,5,5 trimethyl hexanoyl oxybenzene sulphonate | 55.8% |
| Sodium 3,5,5 trimethyl hexanoate | 8.2% |
| 3,5,5 trimethyl hexanoic acid | 0.7% |
| Sodium phenol sulphonate | 2.8% |
| Tallow alcohol $(EO)_{80}$ | 26.7% |
| Water & Misc. | 5.8% |

The reaction yield based on the phenol sulphonate salt conversion was 86.8%.

Example 6

One stage preparation of sodium 3-phenyl propionyl oxybenzene sulphonate using sodium acetate catalyst.

A similar procedure and apparatus to that employed in Example 5 was used with the following quantities of reactants.

| | |
|---|---|
| 3 phenyl propionic acid (hydrocinnamic acid) (MWT 150.2) | 135.18 g (0.9 mole) |
| Acetic anhydride | 36.75 g. (0.36 mole) |
| Sodium phenol sulphonate | 58.85 g (0.3 mole) |
| Sodium acetate (anhydrous) | 3.0 g. |

The hydrocinnamic acid, acetic anhydride and sodium acetate were added to the reaction flask and formed a colourless liquid at 60°C. Sodium phenol sulphonate was added and dispersed by means of agitation and the temperature was increased to 65°C using nitrogen blanketing to prevent oxidation. The contents were refluxed for 4¾ hours before the apparatus was converted to a distillation mode. In the distillation mode the flask contents were allowed to reach a temperature of 260°C over a period of 45 minutes and 80.2 g of mixed acetic acid and hydrocinnamic acid removed as distillate from the flask. The remaining flask contents were cooled to ambient temperature dispersed in diethyl ether and filtered to give 97.88 g of a white crystalline product containing 90% by weight sodium 3-phenyl propionyl oxybenzene sulphonate and 1.2% sodium phenol sulphonate, the remainder being a mixture of 3-phenyl propionic acid and its sodium salt.

**Claims**

1. A method of preparing a $C_6$—$C_{18}$ acyloxybenzene sulphonate salt comprising the steps of
a) forming and maintaining a uniformly dispersed mixture of a $C_2$—$C_3$ acyloxybenzene sulphonate salt in a liquid substituted or unsubstituted $C_6$—$C_{18}$ aliphatic carboxylic acid medium, the molar ratio of carboxylic acid to sulphonate salt being in excess of 1:1 and the sulphonate salt being in particulate form;
b) raising the temperature of the mixture to a value sufficient to cause an acidolysis reaction to take place and to allow the volatilisation and removal of by product $C_2$—$C_3$ alkanoic acid; and
c) recovering the $C_6$—$C_{18}$ acyloxybenzene sulphonate salt.

2. A method according to Claim 1 wherein the molar ratio of carboxylic acid to sulphonate salt is at least 2:1.

3. A method according to either one of Claims 1 and 2 wherein the molar ratio of carboxylic acid to sulphonate salt is at least 3:1, preferably from 4:1 to 6:1.

4. A method according to any one of Claims 1—3 wherein the aliphatic carboxylic acid medium is a non linear carboxylic acid containing a linear chain of at least 5 carbon atoms extending from and including the carbonyl carbon.

5. A method according to Claim 4 wherein the non linear aliphatic carboxylic acid has a $\log P_{OCT}$ in the range from 1.9 to 4.1.

6. A method according to either one of Claims 4 and 5 wherein the non linear aliphatic carboxylic acid contains from 6 to 12, preferably from 7 to 9 carbon atoms.

7. A method according to any one of the preceding claims wherein a transesterification catalyst is incorporated in the alkanoic acid medium.

8. A method according to Claim 7 wherein the catalyst is a basic catalyst.

9. A method according to Claim 8 wherein the basic catalyst is an alkali metal salt of a weak acid, preferably an alkali metal carboxylate.

10. A method according to any one of the preceding claims wherein the phenol sulphonate salt is an alkali metal salt, preferably sodium.

11. A method according to any one of the preceding claims wherein the $C_2$—$C_3$ acyloxybenzene sulphonate salt is formed in situ.

12. A method according to Claim 11 comprising
a) forming a mixture of a phenol sulphonate salt, and a $C_2$—$C_3$ alkanoic anhydride in a liquid substituted or unsubstituted $C_6$—$C_{12}$ aliphatic carboxylic acid medium, the molar ratio of carboxylic acid to sulphonate salt being in excess of 2:1, the molar ratio of anhydride to sulphonate salt being in excess of 1:1, and the sulphonate salt being in particulate form;
b) heating the mixture under reflux to a temperature in the range 140—180°C until the phenol sulphonate salt has reacted to form a $C_2$—$C_3$ acyloxy benzene sulphonate salt;
c) raising the temperature of the mixture to a value sufficient to permit acidolysis to take place, and to allow volatilisation and removal of byproduct $C_2$—$C_3$ alkanoic acid; and
d) recovering the $C_6$—$C_{12}$ acyl oxybenzene sulphonate salt.

13. A method according to any one of the preceding claims wherein the recovery comprises the further steps of forming a dispersion of the completed reaction mixture in a hydrophobic solvent and thereafter separating the precipitated acyloxybenzene sulphonate salt from the supernatant liquor.

14. A method according to Claim 13 wherein the supernatant liquor is heated to volatilise the solvent, and the excess aliphatic carboxyl acid is recycled to step a).

15. A method according to any one of Claims 1—12 wherein the recovery comprises the further steps of
a) forming an intimate dispersion of the completed reaction mixture in a liquified normally solid water

soluble or water dispersible organic binder solid of melting point 40°C, said dispersion taking place under an inert gas atmosphere;

b) cooling said dispersion; and

c) forming said cooled dispersion into solid particulates.

16. A method according to Claim 15 wherein the normally solid water soluble water dispersible organic solid comprises a $C_8$—$C_{18}$ aliphatic alcohol condensed with an average of from 20 to 100 moles of ethylene oxide per mole of alcohol, a polyethylene glycol of molecular weight from 2,000 to 15,000, a $C_{12}$—$C_{18}$ fatty acid, a glyceryl mono $C_{10}$—$C_{14}$ fatty acid ester, or any one or more of the foregoing in admixture with an anionic surfactant.

**Patentansprüche**

1. Ein Verfahren zur Herstellung eines $C_6$—$C_{18}$-Acyloxybenzolsulfonatsalzes, umfassend die Stufen der

a) Bildung und Aufrechterhaltung einer gleichförmig dispersen Mischung eines $C_2$—$C_3$-Acyloxybenzolsulfonatsalzes in einem flüssigen Medium aus substituierter oder unsubstituierter, aliphatischer $C_6$—$C_{18}$-Carbonsäure, wobei das Molverhältnis von Carbonsäure zu Sulfonatsalz mehr als 1:1 beträgt und das Sulfonatsalz in teilchenförmiger Form vorliegt;

b) Erhöhung der Temperatur des Gemisches auf einen Wert, der ausreichend ist, um den Ablauf einer Acidolysereaktion zu bewirken und die Verflüchtigung und Entfernung von Nebenprodukt-$C_2$—$C_3$-Alkansäure zu ermöglichen; und

c) Gewinnung des $C_6$—$C_{18}$-Acyloxybenzolsulfonatsalzes.

2. Ein Verfahren nach Anspruch 1, bei dem das Molverhältnis von Carbonsäure zu Sulfonatsalz wenigstens 2:1 beträgt.

3. Ein Verfahren nach einem der Ansprüche 1 und 2, bei dem das Molverhältnis von Carbonsäure zu Sulfonatsalz wenigstens 3:1, vorzugsweise 4:1 bis 6:1, beträgt.

4. Ein Verfahren nach einem der Ansprüche 1 bis 3, wobei das Medium aus aliphatischer Carbonsäure eine nicht-lineare Carbonsäure ist, welche eine gerade Kette von wenigstens 5 Kohlenstoffatomen enthält, die sich vom Carbonylkohlenstoff erstreckt und diesen einschließt.

5. Ein Verfahren nach Anspruch 4, bei dem die nicht-lineare, aliphatische Carbonsäure einen $\log P_{OCT}$ im Bereich von 1,9 bis 4,1 hat.

6. Ein Verfahren nach einem der Ansprüche 4 und 5, wobei die nicht-lineare, aliphatische Carbonsäure 6 bis 12, vorzugsweise 7 bis 9, Kohlenstoffatome enthält.

7. Ein Verfahren nach einem der vorhergehenden Ansprüche, wobei dem Alkansäuremedium ein Umesterungskatalysator einverleibt ist.

8. Ein Verfahren nach Anspruch 7, wobei der Katalysator ein basischer Katalysator ist.

9. Ein Verfahren nach Anspruch 8, wobei der basische Katalysator ein Alkalimetallsalz einer schwachen Säure, vorzugsweise ein Alkalimetallcarboxylat, ist.

10. Ein Verfahren nach einem der vorhergehenden Ansprüche, wobei das Phenolsulfonatsalz ein Alkalimetallsalz, vorzugsweise Natriumsalz, ist.

11. Ein Verfahren nach einem der vorhergehenden Ansprüche, wobei das $C_2$—$C_3$-Acyloxybenzolsulfonatsalz in situ gebildet wird.

12. Ein Verfahren nach Anspruch 11, umfassend:

a) die Bildung eines Gemisches aus einem Phenolsulfonatsalz und einem $C_2$—$C_3$-Alkansäureanhydrid in einem flüssigen Medium aus substituierter oder unsubstituierter, aliphatischer $C_6$—$C_{12}$-Carbonsäure, wobei das Molverhältnis von Carbonsäure zu Sulfonatsalz mehr als 2:1 beträgt, das Molverhältnis von Anhydrid zu Sulfonatsalz mehr als 1:1 beträgt, und das Sulfonatsalz in teilchenförmiger Form vorliegt;

b) das Erhitzen des Gemisches unter Rückfluß auf eine Temperatur im Bereich von 140—180°C, bis das Phenolsulfonatsalz unter Bildung eines $C_2$—$C_3$-Acyloxybenzolsulfonatsalzes reagiert hat;

c) das Erhöhen der Temperatur des Gemisches auf einen Wert, der ausreichend ist, um den Ablauf von Acidolyse zu gestatten, und die Verflüchtigung und Entfernung von Nebenprodukt-$C_2$—$C_3$-alkansäure zu ermöglichen; und

d) die Gewinnung des $C_6$—$C_{12}$-Acyloxybenzolsulfonatsalzes.

13. Ein Verfahren nach einem der vorhergehenden Ansprüche, wobei die Gewinnung die weiteren Stufen der Bildung einer Dispersion des vervollständigten Reaktionsgemisches in einem hydrophoben Lösungsmittel und anschließend der Trennung des gefällten Acyloxybenzolsulfonatsalzes von der überstehenden Flüssigkeit umfaßt.

14. Ein Verfahren nach Anspruch 13, wobei die überstehende Flüssigkeit zur Verflüchtigung des Lösungsmittels erhitzt wird, und die überschüssige, aliphatische Carbonsäure zur Stufe a) im Kreislauf zurückgeführt wird.

15. Ein Verfahren nach einem der Ansprüche 1 bis 12, wobei die Gewinnung die weiteren Stufen der

a) Bildung einer innigen Dispersion des vervollständigten Reaktionsgemisches in einem verflüssigten, normalerweise festen, wasserlöslichen oder wasserdispergierbaren organischen Bindemittelfeststoffes mit einem Schmelzpunkt von 40°C, wobei die genannte Dispergierung unter einer Inertgasatmosphäre erfolgt;

b) Kühlung der genannten Dispersion; und

c) Verformung der genannten gekühlten Dispersion zu festen Teilchen umfaßt.

16. Ein Verfahren nach Anspruch 15, wobei der normalerweise feste, wasserlösliche oder wasserdispergierbare, organische Feststoff einen aliphatischen $C_8$—$C_{18}$-Alkohol, der mit durchschnittlich 20 bis 100 Mol Ethylenoxid je Mol Alkohol kondensiert ist, ein Polyethylenglykol mit einem Molekulargewicht von 2000 bis 15.000, eine $C_{12}$—$C_{18}$-Fettsäure, einen Glycerylmono-$C_{10}$—$C_{14}$-Fettsäureester, oder irgendeinen oder mehrere der vorstehenden Stoffe im Gemisch mit einem anionischen oberflächenaktiven Mittel umfaßt.

## Revendications

1. Un procédé de préparation d'un sel d'acide acyl $C_6$—$C_{18}$ oxybenzène sulfonique comprenant les étapes consistant à:

a) former et maintenir un mélange uniformément dispersé d'un sel d'acide acyl $C_2$—$C_3$ oxybenzène sulfonique dans un milieu d'acide carboxylique aliphatique en $C_6$ à $C_8$, substitué ou non substitué, liquide, le rapport molaire de l'acide carboxylique au sel d'acide sulfonique étant supérieur à 1:1 et le sel d'acide sulfonique se trouvant sous une forme particulaire;

b) Elever la température du mélange jusqu'à une valeur suffisante pour que se produise une réaction d'acidolyse et pour permettre la volatilisation et l'élimination de l'acide alcanoïque en $C_2$—$C_3$ en tant que produit secondaire; et

c) Récupérer le sel d'acide acyl $C_6$—$C_{18}$ oxybenzène sulfonique.

2. Procédé selon la revendication 1, dans lequel le rapport molaire de l'acide carboxylique au sel d'acide sulfonique est d'au moins 2:1.

3. Procédé selon l'une ou l'autre des revendications 1 et 2, dans lequel le rapport molaire de l'acide carboxylique au sel d'acide sulfonique est d'au moins 3:1, de préférence compris entre 4:1 et 6:1.

4. Procédé selon l'une des revendications 1 à 3, dans lequel le milieu d'acide carboxylique aliphatique est un acide carboxylique non linéaire contenant une chaîne linéaire d'au moins 5 atomes de carbone à partir et y compris le carbone du carbonyle.

5. Procédé selon la revendication 4, dans lequel l'acide carboxylique aliphatique non linéaire possède un $logP_{OCT}$ compris entre 1,9 et 4,1.

6. Procédé selon l'une ou l'autre des revendications 4 et 5, dans lequel l'acide carboxylique aliphatique non linéaire contient 6 à 12, de préférence 7 à 9 atomes de carbone.

7. Procédé selon l'une des revendications précédentes, dans lequel un catalyseur de transestérification est incorporé au milieu d'acide alcanoïque.

8. Procédé selon la revendication 7, dans lequel le catalyseur est un catalyseur basique.

9. Procédé selon la revendication 8, dans lequel le catalyseur basique est un sel de métal alcalin d'un acide faible, de préférence un carboxylate de métal alcalin.

10. Procédé selon l'une des revendications précédentes, dans lequel le sel d'acide phénol sulfonique est un sel de métal alcalin, de préférence de sodium.

11. Procédé selon l'une des revendications précédentes, dans lequel le sel d'acide acyl $C_2$—$C_3$ oxybenzène sulfonique est formé in situ.

12. Procédé selon la revendication 11, consistant à:

a) former un mélange d'un sel d'acide phénol sulfonique et d'un anhydride alcanoïque en $C_2$—$C_3$ dans un milieu d'acide carboxylique aliphatique en $C_6$ à $C_{12}$, substitué ou non substitué, liquide, le rapport molaire de l'acide carboxylique au sel d'acide sulfonique étant supérieur à 2:1, le rapport molaire de l'anhydride au sel d'acide sulfonique étant supérieur à 1:1 et le sel d'acide sulfonique se présentant sous forme particulaire;

b) chauffer le mélange au reflux à une température comprise entre 140 et 180°C jusqu'à ce que le sel d'acide phénol sulfonique ait réagi pour former un sel d'acide acyl $C_2$—$C_3$ oxybenzène sulfonique;

c) élever la température du mélange jusqu'à une valeur suffisante pour permettre l'acidolyse ainsi que la volatilisation et l'élimination de l'acide alcanoïque en $C_2$—$C_3$ en tant que sous-produit; et

d) récupérer le sel d'acide acyl $C_6$—$C_{12}$ oxybenzène sulfonique.

13. Procédé selon l'une des revendications précédentes, dans lequel la récupération comprend les étapes supplémentaires consistant à former une dispersion du mélange réactionnel achevé dans un solvant hydrophobe et ensuite à séparer le sel d'acide acyloxybenzène sulfonique ayant précipité à partir de la liqueur surnageante.

14. Procédé selon la revendication 13, dans lequel on chauffe la liqueur surnageante pour volatiliser le solvant et on recyclique l'acide carboxylique aliphatique en excès vers l'étape a).

15. Procédé selon l'une des revendications 1 à 12, dans lequel la récupération comprend les étapes supplémentaires consistant à:

a) Former une dispersion intime du mélange réactionnel achevé dans un liant organique soluble dans l'eau ou dispersable dans l'eau, liquéfié, normalement solide, d'un point de fusion de 40°C, ladite dispersion ayant lieu sous atmosphère de gaz inerte;

b) Refroidir ladite dispersion; et

c) Transformer ladite dispersion refroidie en des produits particulaires solides.

16. Procédé selon la revendication 15, dans lequel le solide organique soluble dans l'eau ou dispersable dans l'eau, normalement solide, est constitué par un alcool aliphatique en $C_8$ à $C_{18}$ condensé avec une moyenne de 20 à 100 moles d'oxde d'éthylène par mole d'alcool, un polyéthylèneglycol d'un poids moléculaire de 2.000 à 15.000, un acide gras en $C_{12}$ à $C_{18}$, un monoester d'acide gras en $C_{10}$ à $C_{14}$ et de glycérol ou par l'un quelconque ou plusieurs des substances précédentes en mélange avec un agent de surface anionique.